# FASCICULE DE BREVET EUROPEEN

(11) **EP 2 089 405 B1**
(45) Date de publication et mention de la délivrance du brevet: **28.11.2012**
(21) Numéro de dépôt: 07821927.6
(22) Date de dépôt: 26.10.2007
(51) Int. Cl.: C07C 391/00, A23L 1/304

(54) **PROCÉDÉ DE PRÉPARATION DE L'ACIDE 2-HYDROXY-4-MÉTHYLSÉLÉNOBUTYRIQUE, SEUL OU EN MÉLANGE AVEC SON ANALOGUE SOUFRÉ, AINSI QUE LEURS UTILISATIONS EN NUTRITION, EN PARTICULIER EN NUTRITION ANIMALE**
VERFAHREN ZUR HERSTELLUNG VON 2-HYDROXY-4-METHYLSELENOBUTTERSÄURE ALLEIN ODER ALS MISCHUNG MIT IHREM SCHWEFELHALTIGEN ANALOGON, UND ANWENDUNGEN DAVON IN DER ERNÄHRUNG, INSBESONDERE IN DER ERNÄHRUNG VON TIEREN
PROCESS FOR PREPARING 2-HYDROXY-4-METHYLSELENOBUTYRIC ACID, ALONE OR AS A MIXTURE WITH ITS SULPHUR-CONTAINING ANALOGUE, AND USES THEREOF IN NUTRITION, IN PARTICULAR IN ANIMAL NUTRITION

(30) Priorité: 27.10.2006 FR 0609451
(43) Date de publication de la demande: 19.08.2009
(73) Titulaire: Tetrahedron, 94300 Vincennes (FR)
(72) Inventeur: ERDELMEIER, Irène, 75013 Paris (FR); MOUTET, Marc, 94230 Cachan (FR)
(74) Mandataire: Faivre Petit, Frédérique
(86) Numéro de dépôt international: PCT/EP2007/061567
(87) Numéro de publication internationale: WO 2008/049927

(56) Documents cités:
- FR-A1- 2 873 376
- MAY S W: "Selenium-based pharmacological agents: an update" EXPERT OPINION ON INVESTIGATIONAL DRUGS, ASHLEY PUBLICATIONS LTD., LONDON, GB, vol. 11, no. 9, 2002, pages 1261-1269, XP002318375 ISSN: 1354-3784
- AGENAS, L.B.: "The ynthesis of selenium analogues to pantoic acid" ARKIV FOR KEMI, vol. 24, 1965, pages 573-576, XP009083840

## Description

La présente invention concerne un procédé de préparation de l'acide 2-hydroxy-4-méthylsélénobutyrique, seul ou en mélange avec son analogue soufré, ainsi que leurs utilisations en nutrition, en particulier en nutrition animale.

Le sélénium est un micro-nutriment essentiel pour les mammifères et notamment pour l'Homme. Il participe, sous la forme de L(+)-sélénocystéine ou de L(+)-sélénométhionine, à la biosynthèse des sélénoprotéines telles que la Glutathion peroxydase, la Thiorédoxine réductase et la Sélénoprotéine P. Selon la FDA-RDAs, les besoins journaliers en sélénium, chez l'Homme, varient de 10-30µg pour l'enfant, à 40-70µg pour l'adolescent-adulte, ces taux étant particulièrement élevés chez la femme lors de la grossesse (65µg/jour) et lors de la lactation (75µg/jour). La complémentation en L(+)-sélénométhionine (2,7µmoles d'équivalent sélénium) chez la femme allaitant augmente sensiblement la concentration en sélénium dans son lait.

Dans un certain nombre de situations telles que les carences, maladies, ou exposition aux radiations, une complémentation alimentaire en sélénium s'est avérée très bénéfique. C'est tout particulièrement vrai dans le cas d'enfants atteints de maladies génétiques, telles que la phénylcétonurie ou l'hyperphénylalaninémie, qui sont soumis à des régimes alimentaires à faible taux de protéines. Dans un autre domaine, associé à des vitamines, le sélénium sous forme organique telle que la L(+)-sélénométhionine a des effets protecteurs vis à vis des radiations UV chez l'Homme. Enfin, la L(+)-sélénométhionine protège des effets biologiques délétères des radiations ionisantes de haute énergie.

Une demande de brevet français au nom de la demanderesse (FR 2 873376) reporte pour la première fois l'acide 2-hydroxy-4-méthylsélénobutyrique et ses dérivés ainsi que leurs synthèses. Par ailleurs, son analogue soufré, l'acide 2-hydroxy-4-méthylthiobutyrique, encore appelé méthionine liquide, est connu en tant que précurseur de la Méthionine pour l'alimentation animale (WO 9636598). Il est fabriqué industriellement pour cette application à l'échelle de plusieurs centaines de milliers de tonnes par an.

L'acide 2-hydroxy-4-méthylsélénobutyrique, précurseur de la L-Sélénométhionine, ainsi que l'acide 2-hydroxy-4-méthylthiobutyrique, précurseur de la L-méthionine, sont donc des composés d'intérêt majeur en nutrition animale. Il est donc important de pouvoir disposer de procédés de synthèse aisément industrialisables, c'est-à-dire pouvant être réalisés à grande échelle, de la manière la plus simple possible, dont la mise en oeuvre et le coût ne représentent pas des handicaps majeurs.

La Demanderesse a mis au point un procédé de synthèse qui répond à ces critères, et qui présente de nombreux avantages sur les procédés existant.

Ainsi dans un premier aspect, la présente invention concerne un procédé de préparation de l'acide 2-hydroxy-4-méthylsélénobutyrique, caractérisé en qu'il comprend les étapes de :
- réaction du 3-méthylsélénopropionaldéhyde de formule (I) : 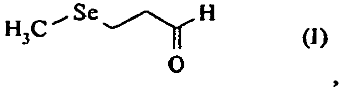 avec un cyanure alcalin de formule M⁺CN⁻, de préférence en présence d'un sel alcalin de bisulfite de formule M⁺HSO₃⁻, M représentant un atome de métal alcalin, dans un solvant protique polaire,
   pour conduire au 2-hydroxy-4-méthylsélénobutyronitrile, composé de formule (II) : 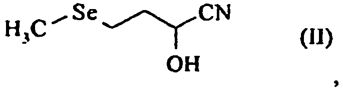
- composé de formule (II) qui est hydrolysé en milieu acide fort concentré, à chaud, dans un solvant protique polaire, pour conduire au composé attendu de formule (IV) : 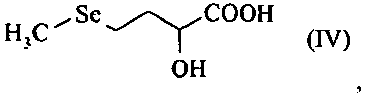
qui peut éventuellement être transformé en un de ses sels après addition avec une base physiologiquement acceptable.

Dans une variante du procédé décrit précédemment, le composé de formule (II) est hydrolysé en milieu acide sulfurique concentré à chaud, dans un solvant protique polaire, pour conduire à un composé de formule (III) : 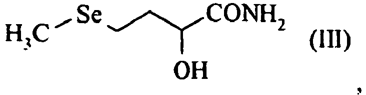 composé de formule (III) qui est lui-même hydrolysé en milieu acide fort concentré à chaud, pour conduire au composé de formule (IV).

Selon une mise en oeuvre avantageuse du procédé selon l'invention, le réactif cyanure alcalin est choisi parmi le cyanure de sodium, le cyanure de potassium, et le cyanure de lithium.

On opère au sein d'un solvant polaire protique tel que par exemple l'eau.

Les réactions subséquentes, permettant de transformer le composé de formule (IV) en ses sels correspondant, sont effectuées dans des conditions classiques, connues de l'homme du métier.

Le procédé selon la présente invention présente l'avantage de ne pas faire appel à des réactifs complexes tels que les composés organométalliques, ni à des agents toxiques tels que les agents alkylants, comme dans les procédés de l'art antérieur (FR 2 573 376). Il est de plus réalisable à grande échelle, dans des solvants non toxiques comme par exemple l'eau. Outre l'aspect faisabilité, ces avantages réduisent aussi considérablement les coûts de mise en oeuvre.

Le composé de formule (J) qui est utilisé comme substrat pour la mise en oeuvre du procédé de l'invention est connu, et son obtention est notamment décrite dans la publication Synthesis, 1988, pages 616-619.

L'intérêt de l'analogue soufré du composé de formule (IV) ci-dessus, l'acide 2-hydroxy-4-méthylthiobutyrique est largement reconnu (voir ci-dessus), en particulier dans le même domaine de la nutrition. L'administration simultanée de ce composé et du composé de formule (IV) présente un avantage indéniable en terme de mise en oeuvre et d'efficacité. Afin de satisfaire les besoins d'une industrialisation, il est important de pouvoir disposer de façon simple de ces deux composés en mélange, et de plus avec un contrôle sur la proportion relative des ces deux dérivés entre eux. Leur administration, simultanée ou de manière séparée, au sein de formulations dans des proportions définies présenterait également un avantage important.

La Demanderesse à démontré que le procédé de préparation selon la présente invention, et décrit précédemment, pouvait être utilisé pour conduire à un mélange des deux composés soufré et sélénié de façon simultanée, ce qui améliore considérablement la productivité industrielle.

Ainsi dans un deuxième aspect, la présente invention concerne un procédé de préparation d'un mélange d'acide 2-hydroxy-4-méthylsélénobutyrique et d'acide 2-hydroxy-4-méthylthiobutyrique, en proportions définies, caractérisé en qu'il comprend les étapes de :
- réaction d'un mélange de 3-méthylsélénopropionaldéhyde de formule (I), et de 3-méthylthiopropionaldéhyde de formule (Ia) : 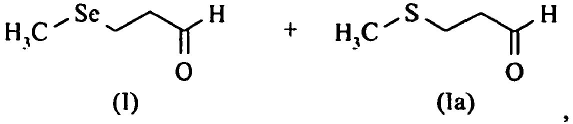 avec un cyanure alcalin de formule M⁺CN⁻, de préférence en présence d'un sel alcalin de bisulfite de formule M⁺HSO₃⁻, M représentant un atome de métal alcalin, dans un solvant protique polaire,
   pour conduire au mélange du 2-hydroxy-4-méthylsélénobutyronitrile de formule (II) et du 2-hydroxy-4-méthylthiobutyronitrile de formule (IIa) : 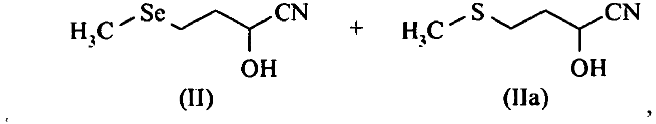
- qui par hydrolyse en milieu acide fort concentré, à chaud, dans un solvant protique polaire, conduit au mélange des deux composés composé attendu de formule (IV) et (IVa) : 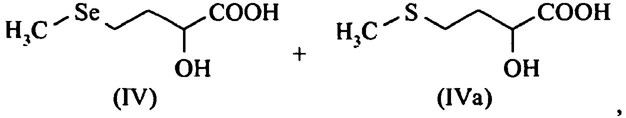
qui peuvent éventuellement être transformés en un mélange de leurs sels après addition avec une base physiologiquement acceptable,
étant entendu que les proportions relatives des deux composés (IV) et (IVa) sont fixées dès le début du procédé, par les quantités relatives des composés (1) et (Ia), et sont conservées tout au long dudit procédé de préparation.

Dans un aspect avantageux, le réactif cyanure alcalin est choisi parmi le cyanure de sodium, le cyanure de potassium, et le cyanure de lithium.

On opère au sein d'un solvant polaire protique tel que par exemple l'eau.

Dans le procédé de l'invention tel que décrit précédemment, la proportion relative des deux composés de formule (IV) et (IVa) est contrôlée et modulable en fonction de l'application envisagée. En particulier, le rapport entre le composé (IV) et le composé (IVa) varie entre 0,01 % et 1,0 % en poids, et préférentiellement entre 0,05 % et 0,5 % en poids.

L'obtention simultanée des composés de formules (IV) et (IVa), précurseurs de sélénométhionine et de méthionine, respectivement, permet aussi d'envisager la préparation de compositions contenant ce mélange, puisque la manipulation en est simplifiée.

Ainsi la présente invention a aussi pour objet une composition, en particulier une composition nutritionnelle comprenant à titre de principe actif un mélange d'acide 2-hydroxy-4-méthylsélénobutyrique de formule (IV) et d'acide 2-hydroxy-4-méthylthiobutyrique de formule (IVa), et un milieu physiologiquement acceptable. Le rapport entre le composé (IV) et le composé (IVa) sera en particulier compris entre 0,01 % et 1,0 % en poids, et préférentiellement entre 0,05 % et 0,5 % en poids.

Par milieu physiologiquement acceptable au sens de la présente invention, on entend un milieu choisi notamment parmi :
* une solution aqueuse, alcoolique ou une huile,
* une émulsion eau/huile ou huile/eau, une microémulsion,
* un gel aqueux,
* une dispersion de vésicules, microcapsules, micro- ou nano-particules,
* un milieu solide composé d'un ou plusieurs additifs et/ou excipients pouvant être sélectionnés parmi des vitamines, des antioxydants naturels, des sels minéraux, des mono-, di- ou polysaccharides, notamment l'acide folique, les vitamines B₆, E ou C, du lactose, de l'amidon. Ce milieu solide composé d'un ou plusieurs additifs et/ou excipients tels que définis ci-dessus, et comprenant au moins l'un des composés de formule générale (I), peut être formulé sous la forme d'une gélule, d'un comprimé ou d'une poudre. Les quantités des différents constituants de ces compositions, autres que le composé de formule (IV) et (IVa), sont celles habituellement utilisées pour les applications citées.

A titre d'exemples non limitatifs donnés simplement à titre d'illustration et qui ne sauraient donc, en aucune façon, limiter la portée de l'invention, ces milieux peuvent être des liquides nutritionnels tels que par exemple du lait alimentaire, des jus de fruits, des sirops, mais aussi du lait pour nourrissons, ou une solution parentérale, du sel de table ou, d'une façon générale, tout aliment complémenté de façon contrôlée en sélénium.

L'invention concerne aussi l'utilisation d'un mélange d'acide 2-hydroxy-4-méthylsélénobutyrique de formule (IV) et d'acide 2-hydroxy-4-méthylthiobutyrique de formule (IVa), en tant qu'ingrédient, complément ou additif alimentaire.

Le composé 2-hydroxy-4-méthylsélénobutyronitrile de formule (II) : 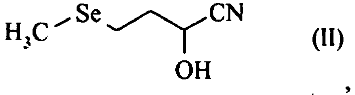 est nouveau, et à ce titre fait partie de l'invention, de même que ses énantiomères.

Au sens de la présente invention, à titre de base physiologiquement acceptable, on peut citer de façon non limitative, les bases minérales telles que les hydroxydes de sodium, de lithium, de calcium, de potassium, de magnésium, d'ammonium, ou de zinc, les carbonates de métaux alcalins ou alcalino-terreux tels que les carbonates et bicarbonates de sodium, de lithium, de calcium, de potassium, de magnésium, d'ammonium ou de zinc, ou des bases organiques comme la méthylamnie, la propylamine, la triméthylamine, la diéthylamine, la triéthylamine, la N,N-diméthyléthanolamine, le tris(hydroxyméthyl)aminoéthane, l'éthanolamine, la pyridine, la picoline, la dicyclohexylamine, la morpholine, la proceïne, la lysine, l'arginine, l'histidine, la N-méthylglucamine, ou encore ses sels de phosphonium tels que les sels d'alkylphosphonium, les sels d'arylphosphonium, les sels d'arylalkylphosphonium, les alkénylarylphosphonium, ou les sels d'ammonium quaternaires tels que les sels de tétra-n-butylammonium.

Le composé de formule (II) est utilisé dans le cadre de la présente invention comme intermédiaire de synthèse dans l'obtention du composé de formule (IV), qui peut être converti en sélénométhionine. Par conséquent l'invention a aussi pour objet l'utilisation du composé de formule (II) telle que définie précédemment, ou de ses énantiomères, en tant que sources de sélénométhionine, et/ou de sélénium chez l'homme ou chez l'animal.

Par ceci on entend notamment l'utilisation dudit composé de formule (II) en tant que :
- précurseurs de L-(+)-sélénométhionine soit directement soit après hydrolyse enzymatique, oxydation et transamination *in vivo ;*
   ou en tant que :
- sources de sélénium, dans le but de pallier une carence partielle ou totale en sélénium;
   ou en tant que
- ingrédients, compléments ou additifs alimentaires pour la fabrication de compositions nutritionnelles pour l'alimentation animale (plus particulièrement les bovins, les ovins, les porcins, les chevaux, les chiens et les chats ainsi que les volailles).

Les exemples qui suivent sont fournis simplement à titre d'illustration et ne sauraient en aucune façon limiter la portée de l'invention.

### Exemple 1 : Préparation du 2-hydroxy-4-(méthylséléno)butyronitrile

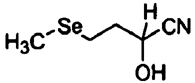

468 mg (3,1 mmol) du 3-méthylsélénopropionaldéhyde (R. Dieden et L. Hevesi, Synthesis 1988, 616-619) sont ajoutés à une solution de 291 mg (2,8 mmol) de bisulfite de sodium dans 1,2 mL d'eau. Le mélange est agité vigoureusement pendant 10 min. à température ambiante, puis on ajoute 155m (3,16 mmol) de cyanure de sodium. Après 2 h d'agitation à température ambiante, on ajoute 5 mL de dichlorométhane, et on décante la phase organique. La phase aqueuse est extraite une deuxième fois avec 5 mL de dichlorométhane. On réunit les phases organiques, puis après séchage (Na₂SO₄), filtration et évaporation, on obtient le composé désiré sous forme d'une huile incolore, qui peut utilisé tel quel dans l'étape suivante.
R_{f} (SiO₂, cyclohexane/acétate d'éthyle, 50/50) : 0.22. ¹H-RMN (CDCl₃, 300 MHz) :
δ (ppm) = 2,06 (s, 3H); 2,27 (m, 2H); 2,73 (m, 2H); 3,20 (sl, 1H, OH); 4,74 (t, J=8 Hz, α-H).
MS (IE, 70eV) : m/z (%) = 179 (80, M^{+●}); 164 (90); 153 (100); 123 (50); 109 (80).

### Exemple 2 : Préparation de l'acide D,L-2-hydroxy-4-méthylsélénobutyrique par hydrolyse du 2-hydroxy-4-(méthylséléno)butyronitrile

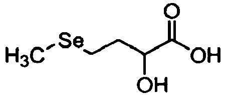

390 mg (2,1 mmol) du composé décrit dans l'exemple 1, sont ajoutés à un mélange de 1,7 mL d'acide chlorhydrique concentré et 3,6 mL d'eau. Le mélange est chauffé sous reflux pendant 6h, puis agité pendant 14h à température ambiante. Ensuite, la phase aqueuse est extraite avec 3x10 mL de terbutylméthyléther. Après séchage (Na₂SO₄), filtration et évaporation, on obtient le composé désiré sous forme d'une huile, qui cristallise à froid.
R_{f} (SiO₂, cyclohexane/acétate d'éthyle, 50/50 + 1% CF₃COOH) : 0.26.
¹H-RMN (CDCl₃, 300 MHz) :
δ (ppm) = 2,02 (s, 3H, SeCH₃); 2,08 (m, 1H); 2,22 (m, 1H); 2,70 (m (sym.), 2H); 4,41 (dd, J = 8 Hz, J = 4 Hz, 1H, α-H).

### Exemple 3: Préparation de l'amide de l'acide D,L-2-hydroxy-4-méthylsélénobutyrique

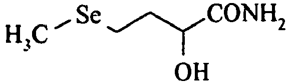

262 mg (1,47 mmol) du 2-hydroxy-4-(méthylséléno)-butyronitrile, décrit dans l'exemple 1, sont ajoutés à un mélange de 0,13 mL d'eau et 0,5 mL d'acide sulfurique concentré. Le mélange est chauffé à 45°C pendant 2 h, puis on ajoute 5 mL d'eau et 5 mL de dichlorométhane. La phase organique est décantée, et la phase aqueuse est extraite une deuxième fois avec 5 mL de dichlorométhane. On réunit les phases organiques, puis après séchage (Na₂SO₄), filtration et évaporation, on obtient 48 mg du composé désiré sous forme d'une huile incolore visqueuse.
R_{f} (SiO₂, dichlorométhane/méthanol, 90/10) : 0.13. ¹H-RMN (CDCl₃, 300 MHz) :
δ (ppm) = 2,06 (s, 3H, SeCH₃); 2,08 (m, 1H); 2,30 (m, 1H); 2,75 (m (sym.), 2H); 3,25 (sl, 1H, OH); 4,36 (dd, J = 8 Hz, J = 4 Hz, 1H, α-H); 5,5 (sl, 1H, NH₂); 6,5 (sl, 1 H, NH₂).
MS (IC, NH₃) : m/z (%) = 215 (40, M+NH₄)⁺; 198 (100, M+H)⁺; 181 (20); 102 (25).

### Exemple 4 : Préparation d'un mélange selon l'invention.

### Etape A : Préparation du mélange du 2-hydroxy-4-(méthylthio)-butyronitrile et du 2-hydroxy-4-(méthylséléno)butyronitrile.

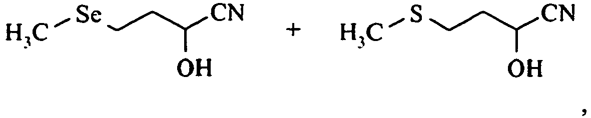

120 mg (0,8 mmol) du 3-méthylséléno-propionaldéhyde (R. Dieden et L. Hevesi, Synthesis 1988, 616-619) et 273 mg (2,5 mmol) du 3-méthylthio-propionaldéhyde sont ajoutés à une solution de 312 mg (3 mmol) de bisulfite de sodium dans 1,2 mL d'eau. Le mélange est agité vigoureusement pendant 10 min. à température ambiante, puis on ajoute 164 mg (3,3 mmol) de cyanure de sodium. Après 2 h d'agitation à température ambiante, on ajoute 5 mL de dichlorométhane, et on décante la phase organique. La phase aqueuse est extraite une deuxième fois avec 5 mL de dichlorométhane. On réunit les phases organiques, puis après séchage (Na₂SO₄), filtration et évaporation, on obtient le mélange de 2-hydroxy-4-(méthylthio)- et (méthylséléno)butyronitrile, dans des proportions 3: 1, sous forme d'une huile jaunâtre, qui peut utilisé tel quel dans l'étape suivante.
¹H-RMN (CDCl₃, 300 MHz) :
δ (ppm) = 2,06 (s, SeCH₃); 2,16 (s, SCH₃), 2,10-2,35 (m); 2,50-2,65 (m); 2,64-2,90 (m); 3,20 (s1, OH); 4,74 (t, J = 8 Hz, 2-H, composé Se); 4,76 (t, J = 8 Hz, 2-H, composé S).

### Etape B : Préparation du mélange de l'acide D,L-2-hydroxy-4-méthylsélénbutyrique et de l'acide D,L-2-hydroxy-4-méthylthiobutyrique :

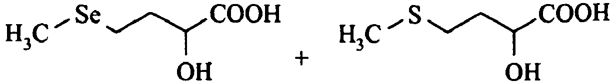

105 mg du produit décrit dans l'étape A, sont ajoutés à un mélange de 0,6 mL d'acide chlorhydrique concentré et 1,3 mL d'eau. Le mélange est chauffé sous reflux pendant 5h, puis la phase aqueuse est extraite avec 2x10 mL de terbutylméthyléther. Après séchage (Na₂SO₄), filtration et évaporation, on obtient le mélange des acides 2-hydroxy-4-(méthylthio)- et (méthylséléno)butyrique dans des proportions 3 : 1, sous forme d'une huile jaune.

| | |
|---|---|
| ES (LC-MS) : | t_{R} = 1,69 min. : 148.9 (M[C5H10O3S]-H⁺); |
| | t_{R} = 2,24 min. : 196,8 (M[C5H10O3Se]-H⁺);. |

### Exemple 5 : Préparation de compositions selon l'invention.

### Exemple 5.A : On a préparé de manière classique des gélules selon la composition suivante :

| | |
|---|---|
| Acide L-2-hydroxy-4-méthylsélénobutyrique | 0,2 mg |
| Acide L-2-hydroxy-4-méthylthiobutyrique | 200 mg |
| Excipients* et enveloppe** | qsp une gélule de 1000 mg |

| | |
|---|---|
| (* amidon de maïs, lactose, stéarate de magnésium, lauryl sulfate de sodium, ** gélatine, dioxyde de titane, colorants). | |

### Exemple 5.B : On a préparé de manière classique des gélules selon la composition suivante :

| | |
|---|---|
| Acide L-2-hydroxy-4-méthylsélénobutyrique | 0,05 mg |
| Acide L-2-hydroxy-4-méthylthiobutyrique | 50 mg |
| Excipients* et enveloppe** | qsp une gélule de 1000 mg |

| | |
|---|---|
| (* amidon de maïs, lactose, stéarate de magnésium, lauryl sulfate de sodium, ** gélatine, dioxyde de titane, colorants). | |

### Exemple 5.C : On a préparé de manière classique des solutions aqueuses selon la composition suivante :

| | |
|---|---|
| Acide L-2-hydroxy-4-méthylsélénobutyrique | 10 mg |
| Acide L-2-hydroxy-4-méthylthiobutyrique | 10 g |
| Eau | qsp 1000ml |

## Revendications

1. Procédé de préparation de l'acide 2-hydroxy-4-méthytsélénobutyrique, caractérisé en qu'il comprend les étapes de :
- réaction du 3-méthylsélénopropionaldehyde de formule (1) : 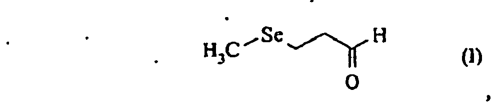 avec un cyanure alcalin de formule M⁺CN⁻, de préférence en présence d'un sel alcalin de bisulfite de formule M⁺HSO₃⁻, M représentant un atome de métal alcalin, dans un solvant protique polaire,
pour conduire au 2-hydroxy-4-méthylsélénobutyronitrile, composé de formule (II) : 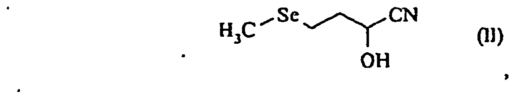
- composé de formule (II) qui est hydrolysé en milieu acide fort concentré, à chaud, dans un solvant pratique polaire, pour conduire au composé attendu de formule (IV) : 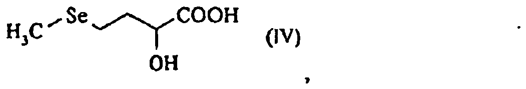 qui peut éventuellement être transformé en un de ses sels après addition avec une base physiologiquement acceptable.

2. Procédé de préparation selon la revendication 1. **caractérisé en ce que** le composé de formule (II) est hydrolysé en milieu acide sulfurique concentré à chaud, dans un solvant protique polaire, pour conduire à un composé de formulé (III) **:** 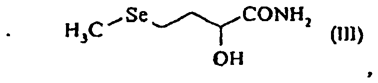 composé de formule (III) qui est lui-même hydrolysé en milieu acide fort concentré à chaud, pour conduire au composé de formule (IV).

3. Procédé selon l'une quelconque des revendications 1 ou 2, **caractérisé en ce que** le cyanure alcalin est choisi parmi le cyanure de sodium, le cyanure de potassium, et le cyanure de lithium.

4. Procédé de préparation selon l'une quelconque des revendications 1 à 3, **caractérisé en ce que** le solvant protique polaire est l'eau.

5. Procédé de préparation d'un mélange d'acide 2-hydroxy-4-méthylsélénobutyrique et d'acide 2-hydroxy-4-méthylthiobutyrique caractérisé en qu'il comprend les étapes de :
- réaction d'un mélange de 3-méthylsélénopropionaldéhyde de formule (I), et de 3-méthylthiopropionaldéhyde de formule (Ia) : 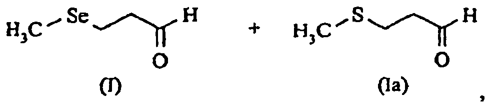 avec un cyanure alcalin de formule M⁺CN⁻, de préférence en présence d'un sel alcalin de bisulfite de formule M⁺HSO₃⁻, M représentant un atome de métal alcalin, dans un solvant protique polaire,
pour conduire au mélange du 2-hydroxy-4-méthylsélénobutyronitrile de formule (II) et du 2-hydroxy-4-méthylthiobutyronitrile de formule (IIa) : 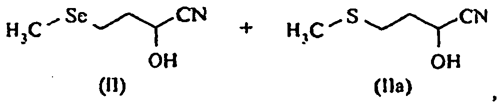
- qui par hydrolyse en milieu acide fort concentré, à chaud, dans un solvant protique polaire, conduit au mélange des deux composés attendus de formule (IV) et (IVa) : 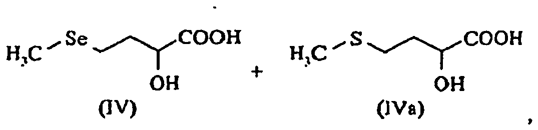 qui peuvent éventuellement être transformés en un mélange de leurs sels après addition avec une base physiologiquement acceptable,
étant entendu que les proportions relatives des deux composés (IV) et (IVa) sont fixées dès le début du procède, par les quantités relatives des composés (I) et (Ia), et sont conservées tout au long dudit procédé de préparation.

6. Procédé selon la revendication 5, **caractérisé en ce que** le cyanure alcalin est choisi parmi le cyanure de sodium, le cyanure de potassium, et le cyanure de lithium.

7. Procécé de préparation selon l'une quelconque des revendications 5 ou 6, **caractérisé en ce que** le solvant protique polaire est l'eau.

8. Procédé de préparation selon l'une quelconque des revendications 5 à 7, **caractérisé en ce que** le rapport entre le composé (IV) et le composé (IVa) varie entre 0,01 % et 1,0 % en poids, préférentiellement entre 0,05 % et 0,5 % en poids.

9. Composé 2-hydroxy-4-méthylsélénobutyronitrite de formule (II) : 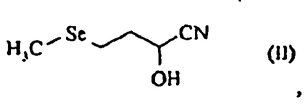 ainsi que ses énantiomères

10. Utilisation du composé de formule (II) telle que définie dans la revendication 9, ou de l'un de ses énantiomères, en tant que sources de sélénométhionine, et/ou de sélénium chez l'homme ou chez l'animal.

11. Composition nutritionnelle comprenant à titre de principe actif un mélange d'acide 2-hydroxy-4-méthylsélénobutyriquc de formule (IV) et d'acide 2-hydroxy-4-méthylthiobutyrique de formule (IVa), et un milieu physiologiquement acceptable.

12. Composition selon la revendication 1 J, **caractérisée en ce que** le rapport entre le composé (IV) et le composé (IVa) varie entre 0,01 % et 1,0 % en poids, préférentiellement entre 0,05 % et 0,5 % en poids.

13. Utilisation d'un mélange d'acide 2-hydroxy-4-méthylsélénobutyrique de formule (IV) et d'acide 2-hydroxy-4-méthylihiobutyrique de formule (IVa), en tant qu'ingrédient, complément ou additif alimentaire.

## Claims

1. A method for preparing 2-hydroxy-4-methylselenobutyric acid, **characterised in that** it comprises the steps of:
■ reacting 3-methylselenopropionaldehyde of formula (I): 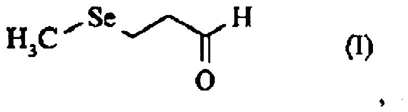 with an alkaline cyanide of formula M⁺CN⁻, preferably in the presence of an alkaline bisulphite salt of formula M⁺HSO₃⁻, M representing an alkali metal atom, in a polar protic solvent,
in order to lead to 2-hydroxy-4-methylselenobutyronitrile, a compound of formula (II): 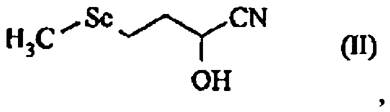
■ a compound of formula (II) which is hydrolysed in a hot concentrated strong acid medium, in a polar protic solvent, in order to lead to the expected compound of formula (IV): 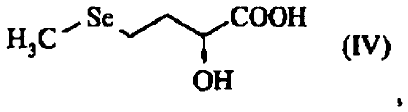 which may optionally be converted into one of the salts thereof after addition with a physiologically acceptable base.

2. The preparation method according to claim 1, **characterised in that** the compound of formula (II) is hydrolysed in a hot concentrated sulphuric acid medium in a polar protic solvent, in order to lead to a compound of formula (III): 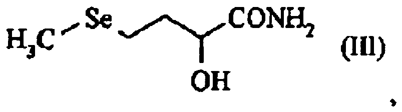 a compound of formula (III) which is in turn hydrolysed in a hot concentrated strong acid medium in order to lead to the compound of formula (IV).

3. The method according to any of claims 1 or 2, **characterised in that** the alkaline cyanide is selected from sodium cyanide, potassium cyanide, and lithium cyanide.

4. The preparation method according to any of claims 1 to 3, **characterised in that** the polar protic solvent is water.

5. A method for preparing a mixture of 2-hydroxy-4-methylselenobutyric acid and of 2-hydroxy-4-methylthiobutyric acid, **characterised in that** it comprises the steps of:
■ reacting a mixture of 3-methylselenopropionaldehyde of formula (I), and of 3-methylthiopropionaldehyde of formula (Ia): 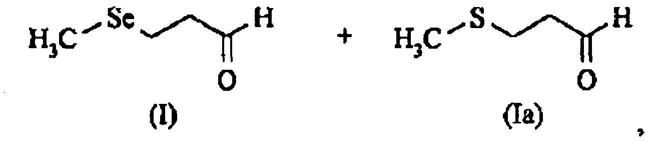 with an alkaline cyanide of formula M⁺CN⁻, preferably in the presence of an alkaline bisulphite salt of formula M⁺HSO₃⁻, M representing an alkali metal atom, in a polar protic solvent, in order to lead to the mixture of 2-hydroxy-4-methylselenobutyronitrile of formula (II) and of 2-hydroxy-4-methylthiobutyronitrile of formula (IIa): 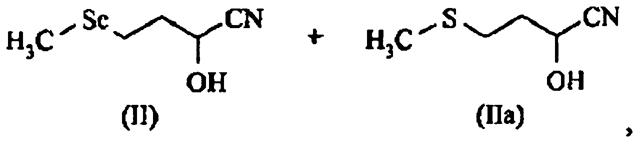
■ which, by hydrolysis in a hot concentrated strong acid medium, in a polar protic solvent, leads to the mixture of the two, expected compounds of formulae (IV) and (IVa): 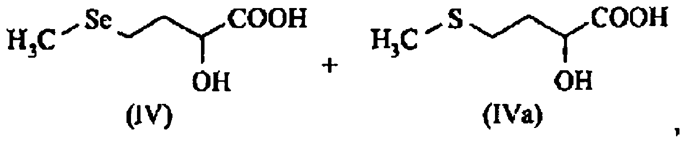 which may optionally be converted into a mixture of the salts thereof after addition with a physiologically acceptable base,
it being understood that the relative proportions of both compounds (IV) and (IVa) are set from the beginning of the method by the relative amounts of compounds (I) and (Ia), and are retained throughout said preparation method.

6. The method according to claim 5, **characterised in that** the alkaline cyanide is selected from sodium cyanide, potassium cyanide and lithium cyanide.

7. The preparation method according to any of claims 5 or 6, **characterised in that** the polar protic solvent is water.

8. The preparation method according to any of claims 5 to 7, **characterised in that** the ratio between the compound (IV) and the compound (IVa) varies between 0.01% and 1.0% by weight, preferentially between 0.05% and 0.5% by weight.

9. The compound, 2-hydroxy-4-methylselenobutyronitrile of formula (II): 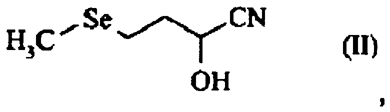 as well as enantiomers thereof.

10. The use of the compound of formula (II) as defined in claim 9, or one of its enantiomers, as sources of selenomethionine and/or of selenium in humans or in animals.

11. A nutritional composition comprising, as an active ingredient, a mixture of 2-hydroxy-4-methylselenobutyric acid of formula (IV) and of 2-hydroxy-4-methylthiobutyric acid of formula (IVa), and a physiologically acceptable medium.

12. The composition according to claim 11, **characterised in that** the ratio between the compound (IV) and the compound (IVa) varies between 0.01% and 1.0% by weight, preferentially between 0.05% and 0.5% by weight.

13. The use of a mixture of 2-hydroxy-4-methylselenobutyric acid of formula (IV) and of 2-hydroxy-4-methylthiobutyric acid of formula (IVa) as a food ingredient, supplement or additive.

## Patentansprüche

1. Verfahren zur Herstellung von 2-Hydroxy-4-methylselenbuttersäure, **dadurch gekennzeichnet, dass** es die Schritte umfasst:
■ Umsetzen von 3-Methylselenpropionaldehyd der Formel (I): 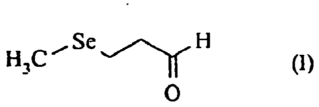 mit einem Alkalicyanid der Formel M⁺CN⁻, vorzugsweise in Gegenwart eines Bisulfit-Alkalisalzes der Formel M⁺HSO₃⁻, wobei M ein Alkalimetallatom bedeutet, in einem polaren protischen Lösungsmittel,
um die Verbindung der Formel (II), 2-Hydroxy-4-methylselenbutyronitril, zu erhalten: 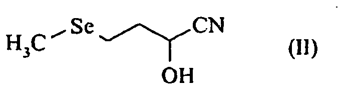
■ wobei die Verbindung der Formel (II) in stark konzentriertem saurem Milieu in der Wärme in einem polaren protischen Lösungsmittel hydrolysiert wird, um die gewünschte Verbindung der Formel (IV) zu erhalten: 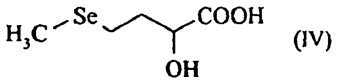 welche gegebenenfalls nach Addition mit einer physiologisch verträglichen Base in eines ihrer Salze umgewandelt werden kann.

2. Verfahren zur Herstellung nach Anspruch 1, **dadurch gekennzeichnet, dass** die Verbindung der Formel (II) in einem konzentrierten Schwefelsäuremilieu in der Wärme in einem polaren protischen Lösungsmittel hydrolysiert wird, um eine Verbindung der Formel (III) zu erhalten: 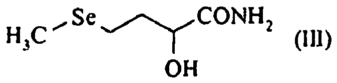 wobei die Verbindung der Formel (III) selbst in stark konzentriertem saurem Milieu in der Wärme hydrolysiert wird, um die Verbindung der Formel (IV) zu erhalten.

3. Verfahren nach einem der Ansprüche 1 oder 2, **dadurch gekennzeichnet, dass** das Alkalicyanid ausgewählt ist aus Natriumcyanid, Kaliumcyanid und Lithiumcyanid.

4. Verfahren zur Herstellung nach einem der Ansprüche 1 bis 3, **dadurch gekennzeichnet, dass** das polare protische Lösungsmittel Wasser ist.

5. Verfahren zur Herstellung eines Gemischs von 2-Hydroxy-4-methylselenbuttersäure und 2-Hydroxy-4-methylthiobuttersäure, **dadurch gekennzeichnet**, das es die Schritte umfasst:
■ Umsetzen eines Gemischs von 3-Methylselenpropionaldehyd der Formel (I) und 3-Methylthiopropionaldehyd der Formel (Ia): 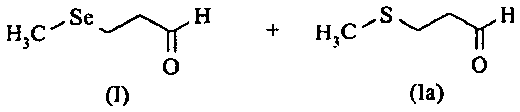 mit einem Alkalicyanid der Formel M⁺CN⁻, vorzugsweise in Gegenwart eines Bisulfit-Alkalisalzes der Formel M⁺HSO₃⁻, wobei M ein Alkalimetallatom bedeutet, in einem polaren protischen Lösungsmittel,
um ein Gemisch von 2-Hydroxy-4-methylselenbutyronitril der Formel (II) und 2-Hydroxy-4-methylthiobutyronitril der Formel (IIa) zu erhalten: 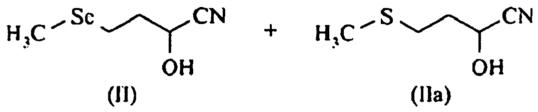
■ welches durch Hydrolyse in stark konzentriertem saurem Milieu in der Wärme in einem polaren protischen Lösungsmittel zu dem Gemisch der beiden gewünschten Verbindungen der Formel (IV) und (IVa) führt: 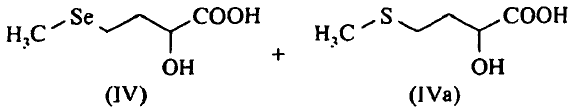 welche nach Addition mit einer physiologisch verträglichen Base gegebenenfalls in ein Gemisch ihrer Salze umgewandelt werden können,
mit der Maßgabe, dass die jeweiligen Anteile der beiden Verbindungen (IV) und (IVa) zu Beginn des Verfahrens durch die jeweiligen Mengen der Verbindungen (I) und (Ia) festgelegt sind und während des gesamten Herstellungsverfahrens beibehalten werden.

6. Verfahren nach Anspruch 5, **dadurch gekennzeichnet, dass** das Alkalicyanid ausgewählt ist aus Natriumcyanid, Kaliumcyanid und Lithiumcyanid.

7. Verfahren zur Herstellung nach einem der Ansprüche 5 oder 6, **dadurch gekennzeichnet, dass** das polare protische Lösungsmittel Wasser ist.

8. Verfahren zur Herstellung nach einem der Ansprüche 5 bis 7, **dadurch gekennzeichnet, dass** das Verhältnis zwischen der Verbindung (IV) und der Verbindung (IVa) zwischen 0,01 und 1,0 Gew.-%, vorzugsweise zwischen 0,05 und 0,5 Gew.-%, liegt.

9. Die Verbindung 2-Hydroxy-4-methylselenbutyronitril der Formel (II): 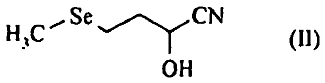 sowie ihre Enantiomere.

10. Verwendung der Verbindung der Formel (II), wie in Anspruch 9 definiert, oder eines ihrer Enantiomere als Selenmethionin- und/oder Selenquellen beim Menschen oder beim Tier.

11. Nahrungszusammensetzung, umfassend als Wirkstoff ein Gemisch von 2-Hydroxy-4-methylselenbuttersäure der Formel (IV) und 2-Hydroxy-4-methylthiobuttersäure der Formel (IVa) und ein physiologisch verträgliches Milieu.

12. Zusammensetzung nach Anspruch 11, **dadurch gekennzeichnet, dass** das Verhältnis zwischen der Verbindung (IV) und der Verbindung (IVa) zwischen 0,01 und 1,0 Gew.- %, vorzugsweise zwischen 0,05 und 0,5 Gew.-%, liegt.

13. Verwendung eines Gemischs von 2-Hydroxy-4-methylselenbuttersäure der Formel (IV) und 2-Hydroxy-4-methylthiobuttersäure der Formel (IVa) als Nahrungsbestandteil, -ergänzungsmittel oder -zusatz.
